# EUROPEAN PATENT SPECIFICATION

(11) **EP 4 731 284 B1**
(45) Date of publication and mention of the grant of the patent: **21.10.2026**
(21) Application number: 24748219.3
(22) Date of filing: 09.07.2024
(51) Int. Cl.: A61M 5/24, A61M 5/315, A61M 5/145

(54) **LEADSCREW TIP FOR AN AUTOINJECTOR**
LEITSPINDELSPITZE FÜR EINEN AUTOINJEKTOR
EMBOUT DE VIS D'ENTRAÎNEMENT POUR UN AUTO-INJECTEUR

(30) Priority: 11.07.2023 US 202363525999 P
(43) Date of publication of application: 29.04.2026
(73) Proprietor: Flextronics AP, LLC, San Jose, CA 95002 (US)
(72) Inventor: SARDO, Giorgio, 20159 Milano (IT)
(74) Representative: FRKelly
(86) International application number: PCT/US2024/037170
(87) International publication number: WO 2025/014929

(56) References cited:
- WO-A1-2023/242830
- US-A1- 2005 015 057
- US-A1- 2009 177 159
- US-A1- 2015 265 776
- US-A1- 2020 206 428
- US-B2- 10 226 582
- US-B2- 11 213 628

## Description

### FIELD OF THE INVENTION

The present disclosure is directed to an autoinjector medical device, and more particularly it is directed to a leadscrew tip for an autoinjector medical device.

### BACKGROUND OF THE INVENTION

An autoinjector medical device is a device that is configured to deliver a dose of a particular drug or medication into a user. Autoinjectors were designed to overcome the hesitation associated with self-administration of needle-based drug delivery. Most autoinjectors utilize one-use, disposable syringes that are prefilled with the drug to be delivered. After the drug has been administered, the syringe can be disposed of and a new prefilled syringe can be inserted into the autoinjector for the next drug delivery. Waste is created due to the disposable syringes used with the autoinjectors, and therefore it is desirable to minimize the waste created by the autoinjectors and disposable syringes where possible.

US 2015/0265776 A1 relates to a torsion spring driven injection device for delivering multiple set doses of a liquid drug from a cartridge by moving a plunger forward inside the cartridge. The torsion spring applies a rotational torque onto a drive member engaging a piston rod which is then driven forward by this rotation. The piston rod driver can be coupled and de-coupled from the torsion spring mechanism. In the de-coupled position, the piston rod driver is freely rotatable and can thus rotate if the piston rod is moved axially. A further torsion arrangement is preferably provided such that a torque is build up between the piston rod driver and the housing whenever the piston rod is moved backwards. This torsion arrangement urges the piston rod driver to rotate in a direction bringing the piston rod forward into abutment with the plunger.

WO 2023/242830 A1 describes a disposable reservoir which includes a plunger head that comprises a PEM and bidirectionally moveable in unison with the PEM between a proximal end of the disposable reservoir and a distal end of the disposable reservoir. The PEM is configured to engage a plunger rod of a reusable part of a drug delivery device. The disposable reservoir also includes a PRM that is configured to release the plunger rod from the PEM upon retraction of the PEM to a stow position in the PRM. The plunger rod may be locked by the PEM and released from the PEM by using a snap-fit feature. Alternatively, the plunger rod may be locked by the PEM by using a snap-fit feature and released from the PEM by using a bayonet connection means. Alternatively, the plunger rod may be both locked by the PEM and released from the PEM by using a bayonet connection means.

US 11,213,628 B2 describes a bearing component which comprises a contact surface inside a periphery, which surrounds a centre, and a coupling feature arranged inside the periphery for rotatably engaging a component of a piston rod perpendicular to the contact surface. The coupling feature includes at least one flexible feature extending from the periphery towards the centre, and the flexible feature is arranged to be deflected towards the periphery by a force exerted on the flexible feature in a direction towards the contact surface and deflected towards the centre by a force exerted on the flexible feature in the opposite direction.

US 2020/0206428 A1 describes a pen-type injection device with a piston rod brake mechanism for improving the determination of an expelled dose.

US 10,226,582 B2 is generally directed to a drug delivery device for selecting and dispensing a number of user variable doses of a medicament comprising a clicker arrangement. The device comprises a housing, a dose selector operable to set a dose by rotation relative to the housing, a number sleeve coupled to the dose selector and arranged within the housing such that at least a portion of the number sleeve is visible through a first aperture in the housing, a piston rod coupled to the housing and to a drive sleeve such that rotation of the drive sleeve relative to the housing causes the piston rod to translate relative to the housing, a clutch arranged between the number sleeve and the drive sleeve, and a dispense clicker comprising a flexible arm and a toothed profile.

US 2005/0015057 A1 discloses a cylindrical piston press foot mounted on a coupling element having a part with an enlarged diameter connected through a part with reduced diameter to the end of a piston rod. The device is useful in injection devices, especially devices by which a liquid medication is apportioned in individual doses from a cartridge.

US 2009/0177159 A1 relates to methods and devices for providing a reduced risk of free flow from medical devices such as an infusion pump. A medicament containing reservoir is provided with a piston having a sealing portion formed by a material of a first stiffness. The piston further comprises a core member of a material having a stiffness larger than said first stiffness, the core member comprising one or more connective members being adapted to cooperate with said one or more protrusions of the linear actuation member. The one or more connective members being adapted to be substantially rigid in the axial direction while being resilient in a radial direction, thereby forming a releasable snap or friction fit connection with the linear actuation member. Thereby a mechanism is provided which is engageable by a purely axial relative displacement and which is disengageable by retracting the linear actuation member axially relative to the piston by exerting a pulling force greater than a predetermined limit.

### SUMMARY OF THE INVENTION

The invention is defined by the subject-matter of claim 1. Further embodiments are defined in the dependent claims.

According to one aspect, an autoinjector medical device is disclosed. The autoinjector medical device can include a housing and a leadscrew disposed within the housing. The leadscrew can be rotatable and axially translatable within the housing along an axis of the leadscrew. The autoinjector medical device can further include a cassette disposed within the housing, the cassette can include a syringe with a needle disposed at an end of the syringe; and a plunger disposed within the syringe, the plunger being axially translatable within the syringe along an axis of the syringe. The autoinjector medical device can further include a leadscrew tip coupled to an end of the leadscrew. The leadscrew tip can include a first end with at least one extension extending away from the first end in a direction parallel to a central axis of the leadscrew tip, a stop extending from the first end into an interior of the leadscrew tip, and a protrusion extending from the at least one extension toward the central axis of the leadscrew tip. The end of the leadscrew coupled to the leadscrew tip can be rotatable within and relative to the leadscrew tip. The leadscrew tip can be configured to engage the plunger to cause the plunger to axially translate within the syringe, and the end of the leadscrew can be disposed axially between the stop and the protrusion.

In one aspect, engagement between the leadscrew tip and the plunger is a non-rotatable engagement, such that the leadscrew tip is rotationally fixed relative to the plunger.

In one aspect, the axis of the leadscrew is axially aligned with the central axis of the leadscrew tip.

In one aspect, the leadscrew tip is coupled to the end of the leadscrew through a snap-fit connection.

In one aspect, the leadscrew tip has a greater outer diameter than an outer diameter of the leadscrew.

In one aspect, the at least one extension includes a plurality of extensions extending from the first end to the second end.

In one aspect, each of the plurality of extensions are circumferentially connected at the first end, and each of the plurality of extensions are circumferentially separated at the second end.

In one aspect, each protrusion extending from each of the at least one extension is oriented perpendicular to the central axis.

In one aspect, each protrusion extending from each of the at least one extension includes an angled surface disposed adjacent the second end of the leadscrew tip, the angled surface being non-parallel and non-perpendicular with respect to the central axis.

In one aspect, the first end of the leadscrew tip has a generally circular cross-sectional shape, with respect to the central axis.

In one aspect, the leadscrew tip is constructed from a low-friction polymeric material.

In one aspect, the leadscrew tip is configured to be coupled to an end of a leadscrew of an autoinjector medical device.

In one aspect, the end of the leadscrew coupled to the leadscrew tip is rotatable within and relative to the leadscrew tip.

In one aspect, a coefficient of friction between the leadscrew tip and the end of the leadscrew is in a range between 0.1 and 0.4.

### BRIEF DESCRIPTION OF THE DRAWINGS

The foregoing Summary as well as the following Detailed Description will be best understood when read in conjunction with the appended drawings, which illustrate a preferred embodiment of the disclosure. In the drawings:
FIG. 1 is a cross-sectional view of a portion of an autoinjector medical device according to the present disclosure.
FIG. 2 is a perspective cross-sectional view of a portion of the autoinjector medical device.
FIG. 3 is a first perspective view of a leadscrew tip of the autoinjector medical device.
FIG. 4 is a second perspective view of the leadscrew tip.
FIG. 5 is a cross-sectional view of the leadscrew tip taken along Section 5-5 in FIG. 3.
FIG. 6 is a cross-sectional view of the leadscrew tip coupled to a leadscrew of the autoinjector medical device.
FIG. 7 is a perspective, partial cross-sectional view of the leadscrew tip coupled to the leadscrew.

### DETAILED DESCRIPTION OF THE PREFERRED EMBODIMENTS

Certain terminology is used in the following description for convenience only and is not limiting. The words "front", "rear", "upper", and "lower" designate directions in the drawings to which reference is made. The words "inwardly" and "outwardly" refer to directions towards and away from parts referenced in the drawings. "Axially" refers to a direction along the axis of an axle, shaft, pin, or the like. A reference to a list of items that are cited as "at least one of a, b, or c" (where a, b, and c represent the items being listed) means any single one of the items a, b, or c, or combinations thereof are included. The terms "about" and "approximately" encompass +/- 10% of an indicated value unless otherwise noted. The term "generally" in connection with a radial direction encompasses +/- 25 degrees. The terminology includes the words specifically noted above, derivatives thereof, and words of similar import.

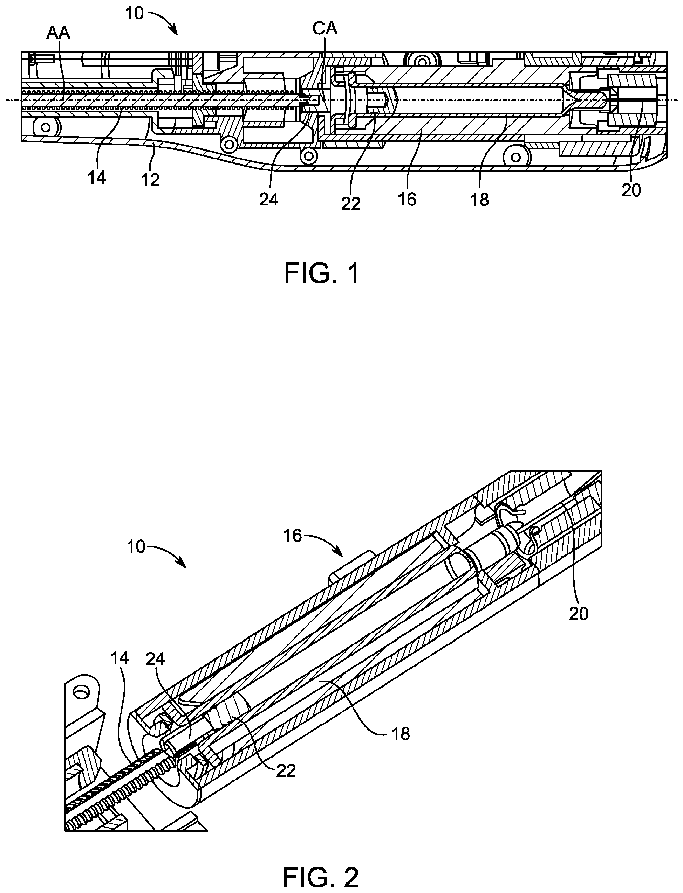

FIG. 1 is a cross-sectional view of a portion of an autoinjector medical device 10. FIG. 2 is a perspective cross-sectional view of a portion of the autoinjector medical device 10. FIGS. 1-2 will be discussed together. Hereinafter the autoinjector medical device 10 will be referred to as the "device 10", but it is to be understood that the autoinjector medical device 10 and the device 10 are referring to the same assembly. The device 10 is configured to deliver a dose of a particular drug or medication into a user, thereby making it easier to self-administer needle-based drugs and medication. Further, the device 10 utilizes one-use, disposable cassettes 16 and syringes 18 that are prefilled with the drug or medication to be delivered. After the drug or medication has been administered, the cassette 16 and the syringe 18 can be disposed of and a new prefilled cassette 16 and syringe 18 can be inserted into the device 10 for the next drug delivery.

The device 10 includes a housing 12 in which the other components of the device 10 are positioned within. Although not shown, it will be appreciated by a person having ordinary skill in the art that the device 10 can include a battery or power source that is utilized to provide electrical energy to a motor or actuator to cause administration of the drug upon the user pressing a button. More specifically, the motor or actuator can be coupled to a leadscrew 14 of the device 10, and the motor or actuator can cause the leadscrew 14 to rotate about an axis AA of the leadscrew 14 which causes the leadscrew 14 to translate axially within the housing 12 along the axis AA of the leadscrew 14. As such, in operation, a user can press a button or switch to activate the motor/actuator, which in turn causes the leadscrew 14 to rotate and translate along the axis AA of the leadscrew 14 to administer and dispense the drug or medication from the device 10, discussed further below.

The device 10 can further include a cassette 16 that can be coupled and fixed within the housing 12 of the device 10, and the cassette 16 can be removed from the device 10 after a drug has been administered. As such, the cassette 16 can be inserted and fixed within the housing 12 for administration of a drug, and then after the drug has been administered the cassette 16 can be removed from the housing 12 and replaced with another cassette 16 having a measured dose of the drug or medication to be administered.

The cassette 16 can include a syringe 18 positioned and coupled within the cassette 16. The syringe 18 can be configured to hold and store the drug or medication that is to be administered or dispensed into the user. Further, the syringe 18 can include a needle 20 disposed and coupled at an end of the syringe 18, with the needle 20 being configured to be inserted into the human body for dispensing the drug or medication from the syringe 18 into the user's body. The syringe 18 can also include a plunger 22 disposed within the syringe 18, and the plunger 22 can be axially translatable within the syringe 18 along an axis of the syringe 18. The plunger 22 can be constructed from a compressible material (e.g. rubber) and the plunger 22 can have an outer diameter slightly larger than an internal diameter of the syringe 18. As such, when the plunger 22 is inserted into the syringe 18, the plunger 22 can slightly radially compress within the internal diameter of the syringe 18 to create a sealing affect to prevent the drug or medication from escaping the syringe 18 at the opposite end of the syringe 18 as the needle 20. Further, the plunger 22 can be forced to translate axially through the syringe 18 to cause the drug or medication within the syringe 18 to dispense from the needle 20.

More specifically, the leadscrew 14 can be rotated to cause the leadscrew 14 to translate axially toward the syringe 18 and plunger 22 of the cassette 16. The leadscrew 14 can translate towards the plunger 22 until an end of the leadscrew 14 contacts the plunger 22 and forces the plunger 22 to translate axially through the syringe 18. An issue that can occur when the end of the leadscrew 14 directly contacts the plunger 22 is that the leadscrew 14 rotating and pressing directly against the plunger 22 can cause the plunger 22 to deform. More specifically, friction between the rotating and pressing leadscrew 14 and the plunger 22 can cause the plunger 22 to twist and deform, which can create a gap or flow path for the drug or medication to leak through in a direction towards the leadscrew 14 and away from the needle 20. The aforementioned is not desirable because it can result in the drug or medication leaking and spilling into the device 10, which can result in an incorrect amount of the drug or medication being dispensed, among other issues not specifically listed.

To remedy the aforementioned issues, a leadscrew tip 24 can be included in the device 10. Specifically, the leadscrew tip 24 can be coupled to the end of the leadscrew 14 that is configured to press against the plunger 22. In some examples, the leadscrew tip 24 can be coupled to the end of the leadscrew 14 through a snap-fit connection. The snap-fit connection allows the leadscrew tip 24 to be coupled to the leadscrew 14 without the need for any further fasteners or components to secure the components together, which results in less overall components in the device 10. The leadscrew tip 24 can be constructed from a low-friction material, such that the end of the leadscrew 14 coupled to the leadscrew tip 24 can rotate within and relative to the leadscrew tip 24 with minimal friction. In some examples, the leadscrew tip 24 can be constructed from a low-friction polymeric material. In further examples, the leadscrew tip 24 can be constructed from a polyoxymethylene thermoplastic (also referred to as POM, acetal polymer, acetal, polyacetal, and polyformaldehyde).

In addition, it is to be understood that the term low-friction used herein refers to the friction that is present between the end of the leadscrew 14 and the leadscrew tip 24 as the leadscrew 14 rotates and presses against the leadscrew tip 24. As such, the material of the leadscrew 14 will affect the friction that is present between the end of the leadscrew 14 and the leadscrew tip 24. In some examples, the leadscrew 14 can be constructed from a steel, stainless steel, or other similar metallic material. In such examples, the value of the coefficient of friction between the end of the leadscrew 14 and the leadscrew tip 24 is preferably in a range between 0.1 and 0.4. Such coefficient of friction values have been determined to minimize and/or eliminate deformation of the plunger 22 during the rotating and pressing contact of the leadscrew 14 on the plunger 22, discussed further below.

Therefore, the device 10 of the present disclosure includes a leadscrew tip 24 that is coupled to the end of the leadscrew 14 positioned closest to the plunger 22 within the syringe 18, and the leadscrew tip 24 is configured to engage the plunger 22 to cause the plunger 22 to translate axially through the syringe 18 while also preventing deformation of the plunger 22. In other words, as the leadscrew 14 rotates and translates axially towards the plunger 22, the end of the leadscrew 14 coupled to the leadscrew tip 24 rotates within and relative to the leadscrew tip 24. Further, the leadscrew tip 24 translates axially with the leadscrew 14 into engagement with the plunger 22, with the engagement between the leadscrew tip 24 and the plunger 22 being a non-rotatable engagement, such that the leadscrew tip 24 is rotationally fixed relative to the plunger 22. As such, the leadscrew tip 24 acts as a buffer or intermediate component that prevents direct contact between the leadscrew 14 and the plunger 22, which prevents the described undesirable deformation of the plunger 22.

As illustrated, the axis AA of the leadscrew 14 is axially aligned with a central axis CA of the leadscrew tip 24. Further, the axes AA, CA of the leadscrew 14 and the leadscrew tip 24, respectively, can be axially aligned with the cassette 16 and the plunger 22 within the syringe 18 of the cassette 16. As such, the leadscrew 14 can translate axially into the syringe 18 to force the plunger 22, through contact with the leadscrew tip 24, in a direction towards the needle 20 to dispense the drug from the syringe 18 into the user. During the axial translation, only the leadscrew tip 24 directly contacts the plunger 22, and therefore any undesirable contact or friction is prevented to aid in the smooth and easy dispensing of the drug from the device 10. To achieve the aforementioned, an outer diameter of the leadscrew tip 24 should preferably be a slightly smaller diameter than an internal diameter of the syringe 18. Further, in some examples, the leadscrew tip 24 can have a greater outer diameter than an outer diameter of the leadscrew 14, such that the leadscrew 14 does not directly contact the syringe 18. In addition, the leadscrew tip 24 should preferably have a slightly smaller outer diameter than the plunger 22 to facilitate smooth translation through the syringe 18.

In operation, a user can press a button/switch to activate the motor/actuator, which in turn causes the leadscrew 14 to rotate and translate along the axis AA of the leadscrew 14 towards the plunger 22. The leadscrew 14 continues to translate towards the plunger 22 until the leadscrew tip 24 contacts the plunger 22 and forces the plunger 22 to translate axially through the syringe 18. During the axial translation of the leadscrew 14, the leadscrew tip 24, and the plunger 22, the end of the leadscrew 14 coupled to the leadscrew tip 24 rotates within the leadscrew tip 24, and the leadscrew tip 24 and the plunger 22 remain rotationally fixed relative to one another. In other words, the leadscrew tip 24 and the plunger 22 do not rotate relative to one another, but rather remain rotationally fixed while translating axially through the syringe 18. The plunger 22 is forced to axially translate through the syringe 18 until the correct/desired amount or dosage of a drug or medication has been dispensed from the needle 20 into the user. The details regarding the shape and structure of the leadscrew tip 24 will be discussed in detail below.

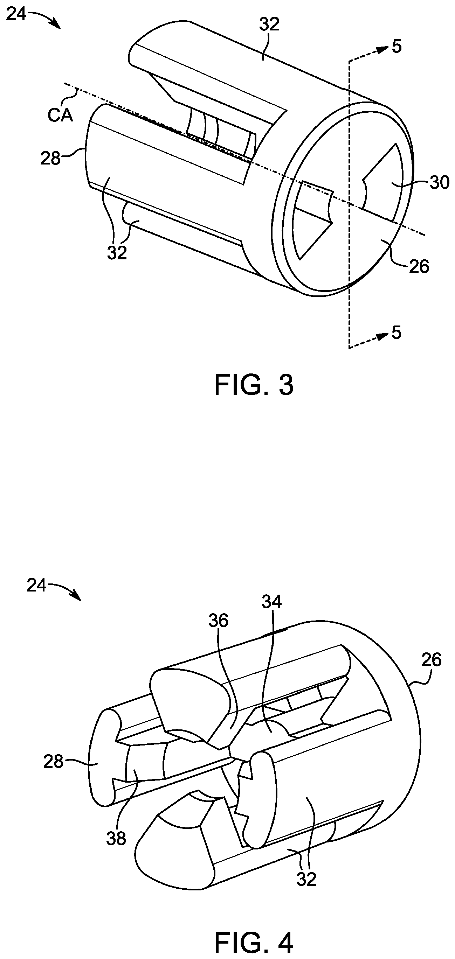

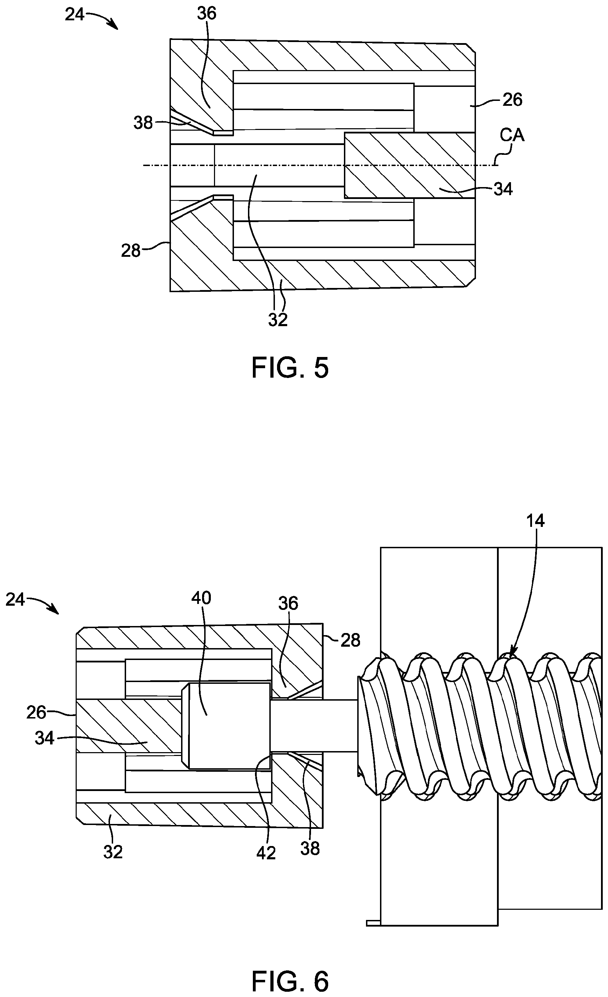

FIG. 3 is a first perspective view of the leadscrew tip 24 of the device 10. FIG. 4 is a second perspective view of the leadscrew tip 24. FIG. 5 is a cross-sectional view of the leadscrew tip 24 taken along Section 5-5 in FIG. 3. FIGS. 3-5 will be discussed together. The leadscrew tip 24 can include a generally cylindrical shape that conforms to an internal shape and diameter of the syringe 18. The leadscrew tip 24 can include a first end 26 and a second end 28 positioned at an opposite end of the leadscrew tip 24 as the first end 26. In some examples, as illustrated, the first end 26 of the leadscrew tip 24 can have a generally circular cross-sectional shape with respect to a central axis CA of the leadscrew tip 24. Further, in some examples, the leadscrew tip 24 can include at least one aperture 30 extending through the first end 26 of the leadscrew tip 24. The at least one aperture 30 can be included to aid in manufacturing the leadscrew tip 24. In other examples, the least one aperture 30 may not be included, depending on the manufacturing technique used to produce the leadscrew tip 24.

In addition, the leadscrew tip 24 can include at least one extension 32 extending from the first end 26 to the second end 28 of the leadscrew tip 24 in a direction generally parallel to the central axis CA of the leadscrew tip 24. In some examples, as illustrated, the leadscrew tip 24 can include a plurality of extensions 32 extending from the first end 26 to the second end 28. In some specific examples, the leadscrew tip 24 can include four extensions 32 extending from the first end 26 to the second end 28. In other examples, the leadscrew tip 24 can include more or less than four extensions 32 extending from the first end 26 to the second end 28. The specific number of extensions 32 can be chosen to achieve the desired clamping or snap-fit connection of the leadscrew tip 24 to the end of the leadscrew 14. Further, it is to be understood that the extensions 32 can also be referred to as "fingers" in some examples. The plurality of extensions 32 are configured to facilitate coupling to the leadscrew 14, and external surfaces of each of the plurality of extensions 32 are configured to aid in guiding the leadscrew tip 24 within and through the syringe 18.

As shown best in FIGS. 3-4, each of the extensions 32 can be circumferentially connected at the first end 26 of the leadscrew tip 24 through the circular-shaped first end 26 extending between and connecting each of the extensions 32. Further, each of the extensions 32 can be circumferentially separated or disconnected at the second end 28 of the leadscrew tip 24 due to the gaps or spaces present between each separate extension 32 at the second end 28. In other words, each of the extensions 32 can be coupled to and extend from the first end 26, and each of the extensions 32 can be cantilevered or free hanging at the second end 28 such that the extensions 32 are not coupled or connected at the second end 28.

The described shape of the extensions 32 (fingers) allows the leadscrew tip 24 to be coupled to the end of the leadscrew 14 without the need for further fasteners or other components. Specifically, when the second end 28 of the leadscrew tip 24 is pressed in an axial direction against the end of the leadscrew 14, each of the extensions 32 bend and flex radially outwards away from the central axis CA of the leadscrew tip 24, allowing the leadscrew tip 24 to slide over the end of the leadscrew 14. Then each of the extensions 32 can return to their original position and snap-fit radially inwards onto the end of the leadscrew 14, discussed in more detail below. As such, each of the extensions 32 can have spring-like characteristics that allow the extensions to flex when inserted onto the end of the leadscrew 14, and then each of the extensions 32 can snap or flex back into their original orientation when inserted far enough onto the end of the leadscrew 14. Although the aforementioned disclosure indicates that each of the extension 32 bend and flex during insertion, it is to be understood that in other examples only some of the extensions 32 will bend and flex during insertion. For example, in some examples only two of the extensions 32 may flex and bend during insertion onto the leadscrew 14, discussed further below.

The leadscrew tip 24 can further include a stop 34 extending from the first end 26 of the leadscrew tip 24, in the same direction as each of the extensions 32, into an interior of the leadscrew tip 24. In addition, the stop 34 can be axially aligned with the central axis CA of the leadscrew tip 24, such that the stop 34 is positioned at an axially central position at one end of the leadscrew tip 24 and the stop 34 can extend toward the interior of the leadscrew tip 24 and towards the second end 28. The stop 34 can be configured as a surface that a distal end of the leadscrew 14 abuts when the leadscrew tip 24 is coupled to the leadscrew 14, preventing the leadscrew 14 from entering further into the leadscrew tip 24, discussed further below.

As shown best in FIG. 5, each of the plurality of extensions 32 can include a protrusion 36 that extends from the respective extension 32 toward the central axis CA of the leadscrew tip 24. Specifically, each protrusion 36 extending from a respective extension 32 can extend from the extension 32 toward an interior of the leadscrew tip 24 at a perpendicular angle with respect to the central axis CA of the leadscrew tip 24. More specifically, an inner edge or surface of each of the protrusions 36 positioned closest to the stop 34 and the first end 26 can be oriented perpendicular to the central axis CA of the leadscrew tip 24. Each protrusion 36 extending from a respective extension 32 can be configured to aid in securing the leadscrew tip 24 to the leadscrew 14, discussed further below. Although the aforementioned disclosure indicates that each of the extension 32 can include a protrusion 36, it is to be understood that in some examples only some of the extensions may include the protrusion 36. For example, in some examples only two of the extensions 32 may include the protrusion 36 extending from the extension 32 toward the central axis CA of the leadscrew tip 24. In addition, in some examples only the extensions 32 including a protrusion 36 may flex and bend during insertion onto the leadscrew 14, discussed further below.

Each protrusion 36 extending from a respective extension 32 can include an angled surface 38 that is disposed adjacent the second end 28 of the leadscrew tip 24. The angled surfaces 38 can each be at a non-parallel and non-perpendicular angle with respect to the central axis CA of the leadscrew tip 24. Further, each angled surface 38 can face in a direction away from the stop 34 and the first end 26. The angled surfaces 38 can be configured to aid in securing and coupling the leadscrew tip 24 to the leadscrew 14. More specifically, the angled surfaces 38 can be configured as lead-in surfaces that aid in the insertion of the leadscrew tip 24 onto an end of the leadscrew 14, discussed further below.

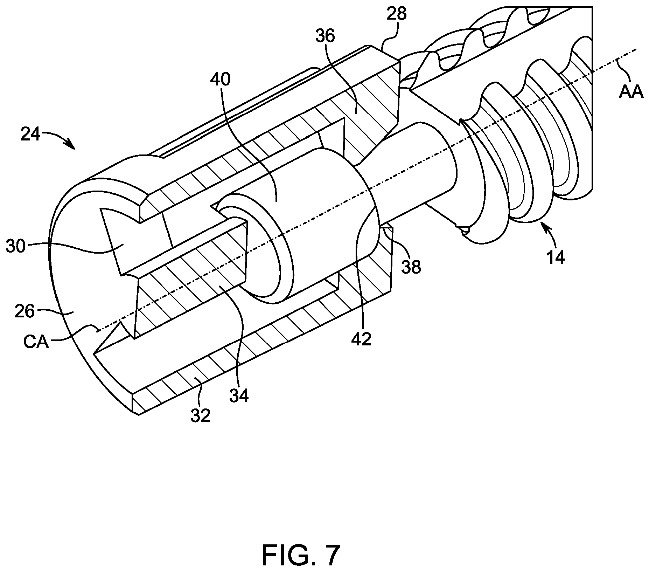

FIG. 6 is a cross-sectional view of the leadscrew tip 24 coupled to the leadscrew 14 of the device 10. FIG. 7 is a perspective, partial cross-sectional view of the leadscrew tip 24 coupled to the leadscrew 14. More specifically, in FIG. 7 the leadscrew tip 24 is illustrated as a cross-section while the leadscrew 14 is not illustrated as a cross-section, to aid in understanding how the leadscrew tip 24 is coupled to the leadscrew 14. FIGS. 6-7 will be discussed together. As discussed, once the leadscrew tip 24 is coupled to the end of the leadscrew 14, the end of the leadscrew 14 can freely rotate within and relative to the leadscrew tip 24. Further, after the leadscrew tip 24 is coupled to the end of the leadscrew 14, the leadscrew tip 24 is intended to remain coupled to the leadscrew 14 for the useful life of the leadscrew 14 and the overall device 10. With that said, it is to be understood that in the event the leadscrew tip 24 is damaged, the leadscrew tip 24 could be removed from the end of the leadscrew 14 and replaced with another leadscrew tip 24.

To couple the leadscrew tip 24 to an end 40 of the leadscrew 14, the leadscrew tip 24 is inserted axially toward the end 40 until the angled surfaces 38 contact the end 40 of the leadscrew 14. The leadscrew tip 24 continues to be forced axially towards the end 40 which causes each of the extensions 32 to flex radially outward away from the axis AA of the leadscrew 14. The extensions 32 flex radially outward until the leadscrew tip 24 has been inserted far enough towards the end 40 that an inner surface 42 of the end 40 is past the innermost surface or edge of the protrusions 36. In addition, the leadscrew tip 24 is inserted far enough onto the end 40 such that a distal end surface of the end 40 abuts or contacts the stop 34 disposed within an interior of the leadscrew tip 24.

As such, the leadscrew tip 24 is inserted onto the end 40 of the leadscrew 14 until the extensions 32 have snapped or otherwise moved back to their original orientation, and the end 40 of the leadscrew 14 is disposed axially between the stop 34 and the protrusions 36, with respect to the central axis CA of the leadscrew tip 24. With the end 40 of the leadscrew 14 disposed axially between the stop 34 and the protrusions 36 of the leadscrew tip 24, the leadscrew tip 24 is fixed axially to the leadscrew 14 such that the leadscrew tip 24 cannot translate axially relative to the leadscrew 14. But due to the structure and connection between the leadscrew 14 and the leadscrew tip 24, the end 40 of the leadscrew 14 is able to rotate about the axis AA of the leadscrew 14 relative to the leadscrew tip 24.

The leadscrew tip 24 allows the leadscrew 14 to rotate and translate axially, while preventing the leadscrew 14 from directly contacting and deforming the plunger 22. As such, the leadscrew tip 24 prevents deformation of the plunger 22 due to the leadscrew tip 24 being rotationally fixed relative to the plunger 22, while allowing the leadscrew 14 to rotate within the leadscrew tip 24. The leadscrew tip 24 is in direct contact with the plunger 22 and prevents direct contact between the plunger 22 and the leadscrew 14 to ensure a drug or medication leakage does not occur around the plunger 22 at the opposite end of the syringe 18 as the needle 20. In addition, the leadscrew tip 24 being part of the device 10 that is non-disposable (i.e. not part of the disposable cassette 16) prevents excess waste and saves money overall based on the fact that the leadscrew tip 24 is not disposed of after each administration of a drug or medication. Although not specifically, those skilled in the art will appreciate the other benefits provided by the leadscrew tip 24 and the overall device 10.

Having thus described the present embodiments in detail, it is to be appreciated and will be apparent to those skilled in the art that many physical changes, only a few of which are exemplified in the detailed description of the disclosure, could be made without altering the inventive concepts and principles embodied therein.

It is also to be appreciated that numerous embodiments incorporating only part of the preferred embodiment are possible which do not alter, with respect to those parts, the inventive concepts and principles embodied therein. The present embodiment and optional configurations are therefore to be considered in all respects as exemplary and/or illustrative and not restrictive, the scope of the disclosure being indicated by the appended claims rather than by the foregoing description, and all alternate embodiments and changes to this embodiment which come within the meaning and range of equivalency of said claims are therefore to be embraced therein.

## Claims

1. An autoinjector medical device (10) comprising:
a housing (12);
a cassette (16) disposed within the housing (12), the cassette (16) including: a syringe (18) with a needle (20) disposed at an end of the syringe (18); and a plunger (22) disposed within the syringe (18), the plunger (22) being axially translatable within the syringe (18) along an axis of the syringe (18);
a leadscrew (14) disposed within the housing (12), the leadscrew (14) being rotatable and axially translatable within the housing (12) along an axis of the leadscrew (14); and
a leadscrew tip (24) coupled to an end (40) of the leadscrew (14), the leadscrew tip (24) comprising:
a first end (26) with at least one extension (32) extending away from the first end (26) in a direction parallel to a central axis of the leadscrew tip (24),
a stop (34) extending from the first end (26) into an interior of the leadscrew tip (24), and a protrusion (36) extending from the at least one extension (32) toward the central axis of the leadscrew tip (24); the end (40) of the leadscrew (14) coupled to the leadscrew tip (24) is rotatable within and relative to the leadscrew tip (24); wherein the leadscrew tip (24) is configured to engage the plunger (22) to cause the plunger (22) to axially translate within the syringe (18); and wherein the end (40) of the leadscrew (14) is disposed axially between the stop (34) and the protrusion (36).

2. The autoinjector medical device (10) of claim 1, wherein engagement between the leadscrew tip (24) and the plunger (22) is a non-rotatable engagement, such that the leadscrew tip (24) is rotationally fixed relative to the plunger (22).

3. The autoinjector medical device (10) of claim 1, wherein the axis of the leadscrew (14) is axially aligned with the central axis of the leadscrew tip.

4. The autoinjector medical device (10) of claim 1, wherein the leadscrew tip (24) is coupled to the end (40) of the leadscrew (14) through a snap-fit connection.

5. The autoinjector medical device (10) of claim 1, wherein the leadscrew tip (24) has a greater outer diameter than an outer diameter of the leadscrew (14).

6. The autoinjector medical device (10) of claim 1, wherein the at least one extension (32) includes a plurality of extensions extending from the first end (26) to the second end (28).

7. The autoinjector medical device (10) of claim 6, wherein the plurality of extensions (32) comprises four extensions.

8. The autoinjector medical device (10) of claim 6, wherein each of the plurality of extensions (32) are circumferentially connected at the first end (26), and each of the plurality of extensions are circumferentially separated at the second end (28).

9. The autoinjector medical device (10) of claim 1, wherein each protrusion (36) extending from each of the at least one extension (32) is oriented perpendicular to the central axis.

10. The autoinjector medical device (10) of claim 1, wherein each protrusion (36) extending from each of the at least one extension (32) includes an angled surface (38) disposed adjacent the second end (28) of the leadscrew tip (24), the angled surface (38) being non-parallel and non-perpendicular with respect to the central axis.

11. The autoinjector medical device (10) of claim 1, wherein the first end (26) of the leadscrew tip (24) has a generally circular cross-sectional shape, with respect to the central axis.

12. The autoinjector medical device (10) of claim 1, wherein the leadscrew tip (24) is constructed from a low-friction polymeric material.

13. The autoinjector medical device (10) of claim 12, wherein the low-friction polymeric material is a polyoxymethylene thermoplastic.

14. The autoinjector medical device (10) of claim 1, wherein the leadscrew (14) is constructed from a metallic material.

15. The autoinjector medical device (10) of claim 1, wherein a coefficient of friction between the leadscrew tip (24) and the end (40) of the leadscrew (14) is in a range between 0.1 and 0.4.

## Patentansprüche

1. Autoinjektormedizinvorrichtung (10), umfassend:
ein Gehäuse (12);
eine Kassette (16), die innerhalb des Gehäuses (12) angeordnet ist, wobei die Kassette (16) beinhaltet: eine Spritze (18) mit einer Nadel (20), die an einem Ende der Spritze (18) angeordnet ist; und einen Kolben (22), der innerhalb der Spritze (18) angeordnet ist, wobei der Kolben (22) innerhalb der Spritze (18) entlang einer Achse der Spritze (18) axial verschiebbar ist;
eine Leitspindel (14), die innerhalb des Gehäuses (12) angeordnet ist, wobei die Leitspindel (14) innerhalb des Gehäuses (12) entlang einer Achse der Leitspindel (14) drehbar und axial verschiebbar ist; und
eine Leitspindelspitze (24), die an ein Ende (40) der Leitspindel (14) gekoppelt ist, wobei die Leitspindelspitze (24) umfasst:
ein erstes Ende (26) mit mindestens einer Verlängerung (32), die sich von dem ersten Ende (26) in einer Richtung parallel zu einer Mittelachse der Leitspindelspitze (24) weg erstreckt,
einen Anschlag (34), der sich von dem ersten Ende (26) in ein Inneres der Leitspindelspitze (24) erstreckt, und einen Vorsprung (36), der sich von der mindestens einen Verlängerung (32) zu der Mittelachse der Leitspindelspitze (24) hin erstreckt; wobei das Ende (40) der Leitspindel (14), das an die Leitspindelspitze (24) gekoppelt ist, innerhalb der und relativ zu der Leitspindelspitze (24) drehbar ist; wobei die Leitspindelspitze (24) zum Eingreifen in den Kolben (22) ausgelegt ist, um zu bewirken, dass der Kolben (22) innerhalb der Spritze (18) axial verschoben wird; und wobei das Ende (40) der Leitspindel (14) axial zwischen dem Anschlag (34) und dem Vorsprung (36) angeordnet ist.

2. Autoinjektormedizinvorrichtung (10) nach Anspruch 1, wobei ein Eingriff zwischen der Leitspindelspitze (24) und dem Kolben (22) ein drehfester Eingriff ist, derart, dass die Leitspindelspitze (24) relativ zu dem Kolben (22) drehfest ist.

3. Autoinjektormedizinvorrichtung (10) nach Anspruch 1, wobei die Achse der Leitspindel (14) mit der Mittelachse der Leitspindelspitze axial ausgerichtet ist.

4. Autoinjektormedizinvorrichtung (10) nach Anspruch 1, wobei die Leitspindelspitze (24) durch eine Schnappverbindung an das Ende (40) der Leitspindel (14) gekoppelt ist.

5. Autoinjektormedizinvorrichtung (10) nach Anspruch 1, wobei die Leitspindelspitze (24) einen größeren Außendurchmesser als ein Außendurchmesser der Leitspindel (14) aufweist.

6. Autoinjektormedizinvorrichtung (10) nach Anspruch 1, wobei die mindestens eine Verlängerung (32) eine Mehrzahl von Verlängerungen beinhaltet, die sich von dem ersten Ende (26) zu dem zweiten Ende (28) erstrecken.

7. Autoinjektormedizinvorrichtung (10) nach Anspruch 6, wobei die Mehrzahl von Verlängerungen (32) vier Verlängerungen umfasst.

8. Autoinjektormedizinvorrichtung (10) nach Anspruch 6, wobei jede der Mehrzahl von Verlängerungen (32) an dem ersten Ende (26) umfangsmäßig verbunden ist, und jede der Mehrzahl von Verlängerungen an dem zweiten Ende (28) umfangsmäßig getrennt ist.

9. Autoinjektormedizinvorrichtung (10) nach Anspruch 1, wobei jeder Vorsprung (36), der sich von jeder der mindestens einen Verlängerung (32) erstreckt, senkrecht zu der Mittelachse ausgerichtet ist.

10. Autoinjektormedizinvorrichtung (10) nach Anspruch 1, wobei jeder Vorsprung (36), der sich von jeder der mindestens einen Verlängerung (32) erstreckt, eine Schrägfläche (38) beinhaltet, die benachbart dem zweiten Ende (28) der Leitspindelspitze (24) angeordnet ist, wobei die Schrägfläche (38) in Bezug auf die Mittelachse nichtparallel und nichtsenkrecht ist.

11. Autoinjektormedizinvorrichtung (10) nach Anspruch 1, wobei das erste Ende (26) der Leitspindelspitze (24) in Bezug auf die Mittelachse eine im Wesentlichen kreisförmige Querschnittsform aufweist.

12. Autoinjektormedizinvorrichtung (10) nach Anspruch 1, wobei die Leitspindelspitze (24) aus einem reibungsarmen Polymermaterial hergestellt ist.

13. Autoinjektormedizinvorrichtung (10) nach Anspruch 12, wobei das reibungsarme Polymermaterial ein thermoplastisches Polyoxymethylen ist.

14. Autoinjektormedizinvorrichtung (10) nach Anspruch 1, wobei die Leitspindel (14) aus einem metallischen Material hergestellt ist.

15. Autoinjektormedizinvorrichtung (10) nach Anspruch 1, wobei ein Reibungskoeffizient zwischen der Leitspindelspitze (24) und dem Ende (40) der Leitspindel (14) in einem Bereich zwischen 0,1 und 0,4 liegt.

## Revendications

1. Dispositif médical auto-injecteur (10) comprenant :
un logement (12) ;
une cassette (16) disposée à l'intérieur du logement (12), la cassette (16) comportant : une seringue (18) avec une aiguille (20) disposée à une extrémité de la seringue (18) ; et
un piston (22) disposé à l'intérieur de la seringue (18), le piston (22) pouvant être déplacé axialement en translation à l'intérieur de la seringue (18) le long d'un axe de la seringue (18) ;
une vis d'entraînement (14) disposée à l'intérieur du logement (12), la vis d'entraînement (14) pouvant tourner et être déplacée axialement en translation à l'intérieur du logement (12) le long d'un axe de la vis d'entraînement (14) ; et
un embout de vis d'entraînement (24) accouplé à une extrémité (40) de la vis d'entraînement (14), l'embout de vis d'entraînement (24) comprenant :
une première extrémité (26) avec au moins une extension (32) s'étendant à l'opposé de la première extrémité (26) dans une direction parallèle à un axe central de l'embout de vis d'entraînement (24),
une butée (34) s'étendant de la première extrémité (26) jusqu'à l'intérieur de l'embout de vis d'entraînement (24), et une protubérance (36) s'étendant à partir de l'au moins une extension (32) vers l'axe central de l'embout de vis d'entraînement (24) ; l'extrémité (40) de la vis d'entraînement (14) accouplée à l'embout de vis d'entraînement (24) pouvant tourner à l'intérieur et par rapport à l'embout de vis d'entraînement (24) ; dans lequel l'embout de vis d'entraînement (24) est configuré pour venir en prise avec le piston (22) afin d'amener le piston (22) à se déplacer axialement en translation à l'intérieur de la seringue (18) ;
et dans lequel l'extrémité (40) de la vis d'entraînement (14) est disposée axialement entre la butée (34) et la protubérance (36).

2. Dispositif médical auto-injecteur (10) selon la revendication 1, dans lequel la mise en prise entre l'embout de vis d'entraînement (24) et le piston (22) est une mise en prise non rotative, de sorte que l'embout de vis d'entraînement (24) est fixe en rotation par rapport au piston (22).

3. Dispositif médical auto-injecteur (10) selon la revendication 1, dans lequel l'axe de la vis d'entraînement (14) est aligné axialement avec l'axe central de l'embout de vis d'entraînement.

4. Dispositif médical auto-injecteur (10) selon la revendication 1, dans lequel l'embout de vis d'entraînement (24) est accouplé à l'extrémité (40) de la vis d'entraînement (14) par l'intermédiaire d'une liaison par encliquetage.

5. Dispositif médical auto-injecteur (10) selon la revendication 1, dans lequel l'embout de vis d'entraînement (24) a un diamètre extérieur supérieur à un diamètre extérieur de la vis d'entraînement (14).

6. Dispositif médical auto-injecteur (10) selon la revendication 1, dans lequel l'au moins une extension (32) comporte une pluralité d'extensions s'étendant à partir de la première extrémité (26) jusqu'à la seconde extrémité (28).

7. Dispositif médical auto-injecteur (10) selon la revendication 6, dans lequel la pluralité d'extensions (32) comprend quatre extensions.

8. Dispositif médical auto-injecteur (10) selon la revendication 6, dans lequel chacune de la pluralité d'extensions (32) est reliée circonférentiellement à la première extrémité (26), et chacune de la pluralité d'extensions est séparée circonférentiellement au niveau de la seconde extrémité (28).

9. Dispositif médical auto-injecteur (10) selon la revendication 1, dans lequel chaque protubérance (36) s'étendant à partir de chacune de l'au moins une extension (32) est orientée perpendiculairement à l'axe central.

10. Dispositif médical auto-injecteur (10) selon la revendication 1, dans lequel chaque protubérance (36) s'étendant à partir de chacune de l'au moins une extension (32) comporte une surface inclinée (38) disposée de manière adjacente à la seconde extrémité (28) de l'embout de vis d'entraînement (24), la surface inclinée (38) étant non parallèle et non perpendiculaire par rapport à l'axe central.

11. Dispositif médical auto-injecteur (10) selon la revendication 1, dans lequel la première extrémité (26) de l'embout de vis d'entraînement (24) a une forme de section transversale généralement circulaire par rapport à l'axe central.

12. Dispositif médical auto-injecteur (10) selon la revendication 1, dans lequel l'embout de vis d'entraînement (24) est construit à partir d'un matériau polymère à faible friction.

13. Dispositif médical auto-injecteur (10) selon la revendication 12, dans lequel le matériau polymère à faible friction est un thermoplastique polyoxyméthylène.

14. Dispositif médical auto-injecteur (10) selon la revendication 1, dans lequel la vis d'entraînement (14) est construite à partir d'un matériau métallique.

15. Dispositif médical auto-injecteur (10) selon la revendication 1, dans lequel un coefficient de friction entre l'embout de vis d'entraînement (24) et l'extrémité (40) de la vis d'entraînement (14) est compris entre 0,1 et 0,4.
